# DEMANDE DE BREVET EUROPEEN

(11) **EP 0 571 296 A1**
(43) Date de publication de la demande: **24.11.1993**
(21) Numéro de dépôt: 93401322.8
(22) Date de dépôt: 24.05.1993
(51) Int. Cl.: A61F 2/16

(54) **Implant porteur d'une lentille intraoculaire**

(30) Priorité: 22.05.1992 FR 9206263
(71) Demandeur: Guilbert, Guillaume, F-14112 Biéville (FR); Guilbert, Bertille, F-14112 Biéville (FR)
(72) Inventeur: Guilbert, Guillaume, F-14112 Biéville (FR); Guilbert, Bertille, F-14112 Biéville (FR)
(74) Mandataire: Dronne, Guy

(57) **Abrégé**

Implant porteur d'une lentille correctrice intra-oculaire, destiné à être introduit dans le sac capsulaire du cristallin sans déformation au travers d'ouvertures beaucoup plus petites que lui, de type notamment disque rigide, comportant une partie optique interne (23), une partie externe haptique (19), et caractérisé en ce qu'il présente des fentes (17-21) ouvertes sur son bord périphérique et allant ainsi délimiter des secteurs d'implant, qui vont être successivement introduits dans l'oeil par une série de manoeuvres, à dominance de rotations anti-horaire et horaire, manoeuvres qui vont être fonction du tracé des fentes. Cet implant est destiné à remplacer le cristallin, dans les interventions pour cataracte.

## Description

La présente invention concerne un implant comportant une lentille correctrice et destiné à être placé à l'intérieur de l'oeil alors que ses dimensions sont supérieures à l'orifice par lequel il va être introduit.Ce dispositif est destiné a être utilisé en chirurgie oculaire, pour remplacer le cristallin, dans le traitement de la cataracte.

La mise en place d'un implant intra-oculaire est un vieux rêve qui a mis longtemps à se concrétiser. Elle est maintenant pratiquée dans la plupart des interventions pour cataracte. Ces implants sont également appelés cristallins artificiels, ou lentilles intra-oculaires bien que cette dernière appellation devrait être réservée à la partie strictement optique. En pratique on décrit à ces lentilles, implants ou cristallins artificiels deux éléments : une partie centrale, I'élément porté, appelée optique, et une partie périphérique, élément porteur, appelée haptique. Cette haptique permet la fixation à l'intérieur de l'oeil où il est essentiel que la lentille soit bien stabilisée, sans quoi surviendront de sérieux ennuis. Ces lentilles procurent une vision confortable, proche de celle d'un oeil normal et sans la moindre sujétion, par rapport aux moyens antérieurement utilisés, puisque la correction, à l'intérieur de l'oeil, est permanente et définitive. Elles sont donc quasi-idéales, en théorie. En pratique, un nombre considérable de travaux et essais divers a déjà été fait, de multiples modèles ont été proposés avec les formes d'haptiques les plus diverses et des modes de fixation variés. Mais inconvénients, difficultés, complications demeurent, constituant un vaste ensemble que l'on va essayer de résumer pour la compréhension des explications qui suivent, relatives à l'intérêt de l'invention.

Ces inconvénients sont liés notamment aux nécessités d'ouverture pour introduire la lentille dans l'oeil, et au système de fixation intra-oculaire.

Cette introduction de la lentille dans l'oeil est effectuée grâce à une incision pratiquée dans la région limbique (à la jonction de la cornée et de la sclère). Cette ouverture est de toute façon nécessaire pour enlever dans un premier temps le cristallin opacifié. On pratique essentiellement à l'heure actuelle des extractions dites extra-capsulaires, c'est-à-dire enlevant une partie de l'enveloppe du cristallin et tout le contenu (cortex et noyau). Ce noyau, de forme circulaire, a ceci de particulier que, passé un certain âge, il devient assez ferme et il ne pourra plus, comme chez l'enfant, être simplement aspiré, ce qui simplifierait bien les choses. Force est donc de l'enlever en une pièce, et comme il mesure selon les cas entre 6 et 9 mm de diamètre, il faut donc faire une incision limbique de ces dimensions, en général de l'ordre de 7 à 8 ou 9 mm (de corde d'arc). Cette extraction, dite manuelle, est à l'heure actuelle de plus en plus remplacée par une fragmentation - destruction - aspiration, le plus souvent grâce à un appareil utilisant les ultrasons (phaco-émulsification), pour laquelle la longueur de l'incision d'ouverture de l'oeil sera minime (2,5 mm), ... puis sera agrandie pour faire entrer l'implant. Bref, l'incision de sortie du cristallin cataracté va également servir à l'introduction de l'implant dans la chambre antérieure de l'oeil. Or, d'une manière générale, plus l'incision sera courte, plus limités seront certains risques de complications ou séquelles cicatricielles, et plus grands seront les avantages (moins de sutures notamment, moins d'astigmatisme postopératoire et meilleur résultat visuel immédiat).

Deuxième difficulté : la fixation intra-oculaire de l'implant. La technique considérée comme la plus recommandable consiste à le placer dans la chambre postérieure de l'oeil, en arrière de l'iris, soit avec appui dans le sillon ciliaire, soit surtout, de préférence, à l'intérieur du sac cristallinien en fixation dite capsulaire ou intra-sacculaire. Autrement dit, l'implant va prendre la place du noyau cristallinien évacué lors de l'extraction extra-capsulaire qui vient d'être évoquée. La technique la meilleure actuellement est de découper une rondelle centrale de capsule antérieure avant d'enlever tout le contenu du cristallin. (Capsules antérieure et postérieure, formant l'enveloppe du cristallin, se réunissent dans la zone appelée équateur du cristallin). Ainsi se trouve réalisée une sorte de bourse, ouverte en avant, de sac capsulaire dans lequel va être placé l'implant correcteur. Le sac du cristallin, également circulaire, a un diamètre d'environ 9 à 10 mm, et une collerette périphérique de capsule antérieure de dimensions suffisantes doit être respectée. (La partie centrale de cette capsule antérieure devrait de toute façon être enlevée car elle s'opacifierait si elle était laissée en place). Ainsi, le diamètre de l'ouverture capsulaire antérieure pourra-t-il être compris entre 4-5 et 7-8 mm. Cette ouverture est pratiquée d'une façon linéaire, régulière et circulaire, - appelée également capsulorhexis. L'avantage de cette façon de procéder est d'obtenir un orifice capsulaire assez résistant tout en étant relativement souple, extensible et déformable ; alors que la moindre petite ébarbure sur le bord de cette ouverture peut entraîner une déchirure s'étendant à l'équateur (avec complications résultantes fâcheuses), à la moindre sollicitation mécanique, - un peu comme pour la cellophane -.

Tous ces détails anatomiques et opératoires sont importants pour la compréhension des explications qui suivent, relatives à l'intérêt de l'invention. Car ces impératifs ont notamment conditionné les différents et très nombreux modèles d'implants jusque-là proposés. Or ces impératifs sont contradictoires : car si l'on souhaite la plus petite incision possible d'ouverture de l'oeil, puis un orifice lui-même assez réduit dans le sac capsulaire où l'on va glisser l'implant, il apparaît dans le même temps qu'il conviendrait que ce sac capsulaire soit le plus complètement possible rempli par l'implant, donc que cet implant soit d'assez grande taille, au mieux d'un diamètre analogue à celui du sac, c'est-à-dire de 9 à 10 mm, circulaire et assez rigide pour bien maintenir tendues les parois du sac. Les capsules restantes vont en effet avoir tendance à s'accoler, à se déformer, se brider partiellement, si elles ne sont pas maintenues tendues. La capsule postérieure pourra se plisser. Les organisations résultantes pourront au fil des mois et années induire des déplacements secondaires de l'implant accolé à telle partie du sac. A cela s'ajoute une évolution, véritable complication propre à l'extraction extra-capsulaire : à la partie périphérique du sac cristallinie subsistent, malgré le nettoyage per-opératoire le plus minutieux, des îlots germinatifs, d'où migreront des cellules qui vont progressivement tapisser la capsule postérieure jusqu'en son centre et donc réduire la vision par constitution d'une sorte de cataracte secondaire derrière l'implant. On peut nettoyer secondairement cette capsule, - mais il faut réintervenir et cela peut récidiver -, ou surtout l'ouvrir, sans opérer cette fois, au laser yag. Mais alors on retrouve un risque plus grand de complications secondaires (rétino-vitréennes surtout) se rapprochant de celles des extractions auparavant pratiquées (dites intra-capsulaires, où on enlevait le cristallin en totalité, avec toutes les capsules). Il convient donc d'essayer de s'opposer à cette migration cellulaire ; or il a été prouvé qu'un obstacle mécanique, sur une capsule tendue, arrêtait généralement la migration des amas cellulaires qui restent ainsi maintenus en périphérie et vont laisser le centre capsulaire transparent.

Toujours en restant strictement dans le cadre des explications qui vont suivre, relatives à l'intérêt de l'invention, on conçoit donc, compte tenu des impératifs parfois contradictoires qui précèdent, que réaliser un implant-lentille intra-oculaire idéal est difficile, et on comprend pourquoi il y a eu déjà tant de modèles proposés. Ils se répartissent schématiquement en plusieurs catégories :
1 - On passera rapidement sur les implants dits souples (optique et haptique), avec des matériaux tels que silicone, héma, etc., pouvant évidemment passer à travers les plus petites incisions. Mais il remplissent mal le sac et n'ont pas les avantages des matériaux rigides que l'on va voir. Ils ont été à ce jour peu employés.
2 - Les implants à optique rigide ont été de très loin les plus utilisés jusqu'alors, et il y a eu pour ces implants deux tendances opposées :
   - Implants rigides (optique et haptique). Très schématiquement leur avantage est de bien remplir le sac, I'inconvénient étant de nécessiter une ouverture large pour l'introduction dans la chambre antérieure ; l'introduction dans le sac capsulaire peut être elle-même délicate, faisant appel à une technique différente et moins bonne que celle de l'ouverture circulaire. Pour atténuer cette difficulté on a proposé des aménagements : encoches, ovalisations, mais il ne s'agit plus alors véritablement d'implants circulaires qui occuperont toute la superficie du sac.
   - Implants plus souples, où seule l'optique est rigide, de taille moyenne à assez grande (diamètre d'environ 7) ou au contraire plus petite (4,5 - 5 mm), permettant donc une introduction dans l'oeil par plus petite incision. Mais si l'optique est donc rigide, on a "compensé" pour l'élément porteur, c'est-à-dire pour l'haptique, en utilisant des combinaisons de matériaux, ou des systèmes d'anses plus ou moins longues et contournées, de spires, "d'amortisseurs-stabilisateurs" variés, qui tous, donc, sont souples, compressibles, permettant une introduction de l'implant dans la chambre antérieure de l'oeil avec une incision à peine plus longue que le diamètre de l'optique en question. (Pour une optique rigide circulaire de 5, on ne peut évidemment faire une incision de longueur inférieure à 5). Ces derniers implants se subdivisent encore en deux grandes familles :
   - Ceux pour lesquels on a cherché à établir une forme et des dimensions les plus adaptées à celles du sac capsulaire : au mieux avec une anse circulaire fermée, souple et donc mince, ou encore avec des anses très enveloppantes, et là encore des dimensions restant de l'ordre de 12 mm, pour que les anses restent légèrement comprimées. Ce sont des modèles moins fréquemment utilisés, car jugés d'une manière générale un peu plus difficiles à mettre en place.
   - Ceux pour lesquels on a cherché la facilité de mise en place : ces modèles ont alors presque tous deux anses souples diamétralement opposées, plus ou moins longues, et assez souvent un diamètre plus grand, de 13 à 14 mm, ce qui permet, si les anses ne sont pas correctement introduites dans le sac, le maintien de toute façon d'une bonne stabilité, la fixation étant alors ciliaire et non plus capsulaire. Ces implants sont de très loin les plus répandus à l'heure actuelle. Toutefois, même si elles sont toutes deux correctement introduites dans le sac, ces anses vont inéluctablement modifier la forme de ce sac, créer des petites tractions capsulaires avec brides, si minimes soient-elles, favoriser des accolements avec éventuelles rétractions secondaires. Car, de deux choses l'une: ou bien elles sont exactement adaptées aux dimensions du sac, elles risquent alors de maintenir insuffisamment l'implant en place, de par leur trop grande souplesse, et elles suivront par ailleurs la tendance imposée par les rétractions capsulaires en ne s'y opposant pas ; ou bien elles sont un peu plus résistantes et de diamètre un peu plus grand, et elles vont alors obligatoirement déformer la périphérie du sac, si peu que ce soit, puisqu'elles ne maintiendront pas une "conformation" véritablement circulaire. Il n'est pas exclu, par ailleurs, qu'étant un peu trop rigides, elles soient, au moins pour l'une d'entre elles, expulsées en quelque sorte du sac, secondairement (comme hors d'un corset trop serré), ce qui entraînera un décentrement secondaire de l'implant. Enfin, même si elles sont faciles à insérer toutes deux dans le sac, l'une d'entre elles peut créer temporairement, au moment de l'introduction,une déformation à la périphérie du sac, mais parfois aussi un traumatisme, avec lésions de l'appareil zonulaire suspenseur du sac à la paroi de l'oeil. Et cela, quelles que soient les précautions prises, car il s'agit d'une obligation mécanique incontournable, liée à la conception et à la forme de ces haptiques, pour certains modèles à anses un peu plus rigides. Toutes ces explications apportées avant d'aborder la description du dispositif propre à l'invention montrent, en résumé, que certaines questions restent non résolues.

Toujours à propos des explications qui vont suivre, relatives à l'intérêt de l'invention, il convient encore d'apporter une précision essentielle. Car l'implant en question, qui va être introduit dans l'oeil, doit évidemment l'être de façon atraumatique. Or, la chambre antérieure de l'oeil, où va d'abord passer l'implant, a des dimensions limitées, et est fermée en périphérie par l'insertion annulaire de l'iris à la paroi du globe. L'ensemble de cette chambre antérieure représente un espace d'environ 14 mm de diamètre, espace dans lequel un implant de diamètre 10 mm va donc devoir entrer sans jamais déborder au-delà de ces limites de 14 mm. Et le limbe chirurgical, où sont pratiquées classiquement les incisions d'ouverture, se situe lui-même à un diamètre de 12 mm, dans la chambre antérieure. Il existe donc seulement, en arrière de ce cercle de 6 mm de rayon, un simple millimètre "de battement" possible, pour les manoeuvres d'introduction, au-delà duquel on bute inexorablement sur les limites de la chambre antérieure, c'est-à-dire l'iris et les structures fragiles de l'angle irido-cornéen.

Le dispositif selon l'invention tient compte de ces impératifs inhérents à l'anatomie de l'oeil et permet de contourner ces deux difficultés majeures et contradictoires précédemment analysées, quant aux dimensions respectives de l'incision puis de l'ouverture du sac capsulaire, beaucoup plus petites que l'implant à mettre en place dans ce sac. Et il s'avère en fait possible d'obtenir cet implant circulaire, suffisamment rigide, de grand diamètre, analogue à celui du sac, de 9 à 10 mm, qui va pouvoir passer à travers une ouverture limbique limitée de 5 voire de 4 mm puis au travers d'une ouverture capsulaire de diamètre 5 à 6 mm tout en occupant ensuite l'entière superficie du sac, - sans parler de l'orifice pupillaire, également circulaire, habituellement bien dilaté "médicamenteusement", pendant ces interventions, mais qui dans certains peut être plus rétréci, de l'ordre de 4 à 6 mm, et devra lui aussi être franchi par l'implant destiné au sac capsulaire.

Cela s'obtient en délimitant sur l'implant des secteurs dont la partie la plus grande ne dépassera pas la dimension de l'ouverture que l'on souhaite pratiquer. Ces secteurs ainsi délimités sont progressivement introduits à l'intérieur de l'oeil, au travers de l'ouverture réduite, grâce à des manoeuvres alternées, à dominante de rotation horaire et anti-horaire. Ces manoeuvres et la délimitation de ces secteurs doivent être tels qu'il n'y ait à aucun moment traumatisme à la périphérie de la chambre antérieure, par rotation et saillie trop marquée de l'un des secteurs déjà introduit à l'intérieur de l'oeil. Des manoeuvres du même ordre, de rotation alternée, permettront l'introduction ensuite dans le sac capsulaire. Cette délimitation de l'implant en secteurs se fait grâce à des fentes étroites permettant juste le glissement entre les lèvres de l'incision pratiquée au limbe. Le tracé de ces fentes est minutieusement établi pour que cette introduction par paliers successifs soit à la fois aisée et atraumatique, et tel que la distance entre la partie la plus interne de chaque fente, si elles sont au nombre de deux, et l'extrémité externe de l'autre ne soit pas supérieure au diamètre de l'optique. Ceci du moins pour une efficacité optimale, c'est-à-dire permettant d'introduire l'implant par la plus petite ouverture possible. Etant entendu que l'existence de ces fentes ne doit pas modifier la cohérence d'ensemble de l'implant circulaire, qui reste d'une seule pièce formant un ensemble suffisamment stable, ferme et même rigide, - aucun des éléments n'étant pliable ou compressible -, pour constituer un disque ayant les qualités morphologiques d'un disque homogène et permettant ainsi d'occuper tout l'espace du sac capsulaire. Etant entendu par ailleurs que ces fentes respectent l'aire centrale, correspondant à la zone optique circulaire, dont le diamètre conditionnera la longueur de l'incision limbique. Un implant circulaire de diamètre 10 mm avec une optique de diamètre 7 mm pourra ainsi être introduit au travers d'une incision limbique de 7 mm. Le même implant de 10, avec une optique de 5 mm voire de 4, pourra être introduit par une ouverture analogue de 5 voire de 4. On est rarement descendu au-dessous d'optiques de 5 ou 4,5. Les chiffres de 4 mm voire de 3,5mm et peut-être moins paraissent envisageables cependant, dès lors que l'implant restera bien centré puisque occupant tout le sac capsulaire et n'ayant plus à subir les rétractions capsulaires secondaires observées avec des implants classiques tenus dans le sac grâce à des anses souples, malléables et donc secondairement modifiables dans leur position. Une telle incision limbique de 4, de 4,5 ou même de 5 mm est en pratique déjà suffisamment limitée pour que soit notablement diminué le nombre des sutures nécessaires pour la fermeture (il peut être réduit à deux ou trois) et surtout le risque d'astigmatisme postopératoire.

PIus précisément, l'invention est définie par la revendication 1.

Les dessins annexés illustrent à titre d'exemple non limitatif plusieurs modes de réalisation du dispositif selon la présente invention. Sur ces dessins :
- La figure 1 est une vue antérieure du globe oculaire sur lequel on a placé un cadran horaire pour indiquer ce que signifient des expressions employées en ophtalmologie telles que ouverture limbique sur 11h ou 1h, et où on a schématisé le tracé d'une incision limbique de 4 mm sur midi.
- La figure 2 est une vue schématique antérieure d'un implant ayant une optique de diamètre 4,8 mm, avec plusieurs types de fentes.
- La figure 3 est une vue schématique antérieure d'un implant ayant une optique de 5 mm, avec deux fentes.
- La figure 4 est une vue schématique antérieure d'un implant ayant une optique de 5mm, avec là encore deux fentes, selon une autre disposition, pour laquelle l'incison devra être de 7 mm, et où le disque plein a été évidé sur une partie de sa périphérie.
- La figure 5 est une vue antérieure d'un implant avec optique de diamètre 5mm et également deux fentes, où ont été représentés par des lettres dans l'ordre successif (A - B - C- ...) les différents segments de l'implant qui vont être successivement introduits dans l'oeil au fil des différentes manoeuvres schématisées par des flèches.

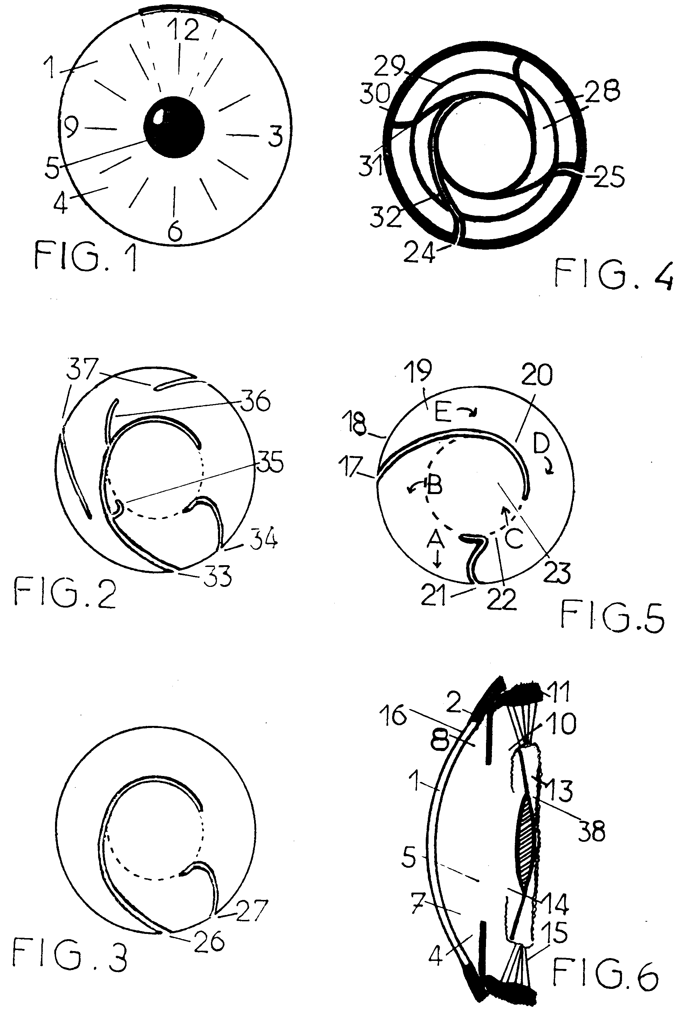

- La figure 6 est une coupe verticale sagittale de la partie antérieure de l'oeil montrant la situation intra-oculaire de l'un des implants des figures 2, 3, 4 ou 5, en place dans le sac capsulaire. Seule la partie antérieure de l'oeil est représentée schématiquement et tous les détails ne sont pas reproduits. On peut se reporter aux manuels classiques pour la description et les planches anatomiques d'un globe oculaire. Les numéros 1 et 2 se rapportent respectivement à la cornée et à la sclère. Le numéro 4 désigne l'iris, le 5 la pupille, le 7 la chambre antérieure de l'oeil, le 8 l'angle irido-cornéen, le 10 la chambre postérieure, le 11 le corps ciliaire, le 13 le sac capsulaire, le 14 son ouverture antérieure, le 15 les fibres de la zonule, le 16 le limbe chirurgical, où s'insère la conjonctive qui recouvre la partie antérieure de la sclère et n'est pas représentée ici.

Le dispositif, objet de l'invention, va maintenant être décrit dans une de ses variantes, dans le cas d'un implant avec une optique de 5mm de diamètre (figure 5). Ensuite va être décrite la manoeuvre de l'introduction de cet implant à travers l'incision limbique puis l'ouverture dans le sac capsulaire. On supposera que l'on regarde de face cet implant présenté comme un cadran horaire. Une longue fente 17 va s'ouvrir dans son bord périphérique 18 sur 9h. Cette fente va suivre un trajet courbe à travers l'haptique 19 et progressivement dirigé vers le centre en haut et à droite, jusqu'à atteindre le bord de l'optique 20. Elle restera ensuite concentrique à cette optique circulaire, dont elle formera en fait la limite jusqu'à 3h30. Une deuxième fente 21 part du bord périphérique de l'implant, sur le méridien de 6h et suivant un trajet légèrement curviligne va rejoindre le bord de la lentille jusqu'à 7h. L'ensemble de ces deux fentes, tracées donc dans un disque plein constitué par exemple d'un polymère transparent ou de verre, et dont la partie centrale 23, correctrice, aura la puissance optique adéquate, reste suffisamment rigide pour conserver à l'ensemble de l'implant sa forme circulaire de façon stable. L'introduction d'un tel implant se fait de la manière suivante. Extraction extra-capsulaire par phaco-émulsification après ouverture circulaire linéaire de la capsule antérieure du cristallin. L'incision limbique de phaco-émulsification est ensuite légèrement agrandie à 5mm pour permettre l'introduction de l'implant, et la chambre antérieure de l'oeil est remplie d'une substance visqueuse transparente à pouvoir tamponnant comme il est de routine. Vont ensuite se succéder les temps suivants :
A - Introduction de la partie se trouvant juste à gauche (si l'on regarde de face) de la petite fente.
B - Rotation anti-horaire de l'implant, la partie la plus interne de la petite fente étant bien calée dans la partie droite de l'incision. Toute l'optique de l'implant va ainsi être introduite dans la chambre antérieure jusqu'à proximité de l'extrémité interne de la grande fente.
C - Légère translation vers la gauche (dans la nouvelle situation ainsi acquise) de l'ensemble de l'implant pour permettre le passage dans la chambre antérieure de la partie de l'implant se trouvant à ce stade encore à l'extérieur de l'oeil et d'abord de la zone comprise entre la petite fente, la partie voisine périphérique de l'optique et celle adjacente de l'haptique, et l'extrémité interne de la grande fente.
D - Le mouvement sera ensuite exclusivement une rotation horaire, douce et progressive, en veillant simplement attentivement à ce que l'implant reste légèrement poussé vers le bas, avec le bord externe de la grande fente bien régulièrement ancré entre les lèvres de l'incision. Cela afin que l'introduction progressive, sur 360°, du restant de l'implant toujours hors de l'oeil (toute la partie périphérique en dehors de la grande fente), se fasse sans que la partie déjà à l'intérieur de la chambre antérieure soit amenée contre les limites de cette chambre antérieure, tout au long de la rotation horaire.
E - Ne persiste ainsi plus à l'extérieur, en fin de manoeuvre, qu'un croissant de lune correspondant à la partie située en dehors de la grande fente, dans sa portion la plus externe. Progressivement ce croissant est introduit dans l'oeil, en surveillant là encore que la partie terminale reste bien appliquée vers le bas, contre l'incision, tout en continuant le mouvement de rotation. Cela afin que la partie déjà introduite de l'implant ne vienne buter dans l'angle irido-cornéen en haut et à gauche. Ainsi l'implant de 10 mm disparaît-il dans la chambre antérieure, par une incison de 5mm, grâce à ces deux fentes et à leur trajet, et grâce aux manoeuvres décrites, à base principalement de rotation anti-horaire puis horaire.

Une fois l'implant dans la chambre antérieure, reste à le faire passer dans le sac capsulaire. Il est pour cela plusieurs solutions. La partie effilée comprise entre la grande fente et la partie périphérique de l'implant, - entrée en dernier dans la chambre antérieure -, est poussée délicatement à l'intérieur puis vers la périphérie du sac capsulaire. Un mouvement progressif de rotation anti-horaire va faire entrer presque toute la périphérie de l'implant dans le sac dont les bords, rappelons-le, sont légèrement déformables et extensibles. L'implant passe ainsi presque spontanément dans le sac. Au besoin une petite poussée inverse avec rotation cette fois horaire l'y introduit complètement. On peut préférer la manoeuvre consistant à faire rentrer d'abord la partie de l'haptique délimitée par la portion externe de la grande fente et la petite fente, après quoi le reste va suivre, par rotation. Ces manoeuvres, qui en théorie seraient plus difficiles si les bords du sac et son équateur étaient aussi rigides que ne l'est l'incision limbique, sont en pratique facilitées par la malléabilité relative, donc, des bords du sac une ouverture de seulement 4 ou 5 mm étant possible, mais pouvant également être plutôt de l'ordre de 6 ou 7, éventuellement avec découpe capsulaire antérieure ovalisée au lieu d'être circulaire.

Une deuxième variante du dispositif selon l'invention va être maintenant décrite où cette fois l'implant a toujours un diamètre de 10 mm et une optique de 5 mm (figure 4), et va être introduit à travers une incision de 7 mm : cas d'une extraction extra-capsulaire manuelle classique si on n'a pas pratiqué de phako-émulsification. L'implant en question comporte là également deux fentes, mais qui ne suivent pas le même trajet. La grande fente 24 part de 6h, à la périphérie, et va, suivant une ligne courbe, rejoindre le bord de l'optique qu'elle atteint un peu avant 9h et suit jusqu'à midi. La petite fente 25 part également du bord périphérique, mais sur 3h, selon un trajet curviligne, s'arrêtant à mi-distance entre la périphérie de l'implant et la périphérie de l'optique. Les deux fentes sont donc moins longues que dans le modèle précédent et l'introduction sera d'autant plus rapide et facile :
A - On commence là encore par introduire la partie à gauche de la petite fente
B - On effectue ensuite la même rotation anti-horaire qui fait passer presque toute la lentille dans la chambre antérieure.
C - On avance légèrement l'ensemble vers la gauche et vers le bas.
D - Pour pouvoir faire ensuite une rotation horaire et introduire le restant de la périphérie de l'implant.
E - En veillant, pour la partie toute terminale du croissant restant à introduire, à bien maintenir l'implant vers le bas et la droite, en continuant la rotation.

Mêmes manoeuvres ensuite pour introduire l'implant dans le sac capsulaire.

Une troisième variante du dispositif selon l'invention est représentée figure 3 où l'implant de 10 mm avec optique de 5 mm pourrait cette fois être introduit avec une incision de 5 mm. Les grande 26 et petite 27 fentes partent toutes deux du bord périphérique, comme pour le modèle de la figure 4 et 5, mais sont plus rapprochées l'une de l'autre. Le mode d'introduction dans l'oeil est identique, de même que dans le sac capsulaire.

Les protocoles qui viennent d'être décrits ne l'ont été qu'à titre indicatif et explicatif, de nombreuses autres façons de procéder étant utilisables en fonction des diverses modifications apportées au dispositif, et cela sans sortir du cadre de l'invention. Certaines de ces modifications vont être maintenant envisagées de façon non limitative. Ainsi les fentes, qui sont indispensables pour l'introduction de l'implant à travers une incision étroite, avec manoeuvres alternées de rotation horaire et anti-horaire, peuvent être variées 33-34-35-36-37 dans leur disposition, leur siège, leur aspect (curviligne, en segments de droite, ...), ou encore dans leur nombre : plusieurs peuvent être par exemple disposées sur une certaine longueur et un trajet non plus centripète mais légèrement divergent, à partir de la partie moyenne et surtout externe de la grande fente, ce qui amènera à introduire la partie terminale de l'implant par une succession de manoeuvres de translation et de rotation, dans un sens puis dans l'autre, au lieu d'effectuer la manoeuvre terminale de rotation d'un seul tenant. Ces fentes, ou l'une d'entre elles, notamment la plus courte, peuvent se développer à partir d'une autre fente et non pas seulement à partir du bord périphérique de l'implant ; ou encore être partiellement "englobées" dans un espace libre pratiqué dans l'haptique. Cet espace libre, plus ou moins important, pourrait être considéré comme à la fois facilitant et gênant : il facilite l'apparente liberté de manoeuvre de l'implant ; et il peut gêner, en rendant plus délicate l'introduction ou plus exactement le déplacement de l'implant à l'intérieur de la chambre antérieure de l'oeil avec sa conséquence vis-à-vis essentiellement des limites de cette chambre, alors que les fentes permettent de mieux rester dans le cadre précis de la manoeuvre d'introduction atraumatique à l'intérieur de l'oeil.

Mais cette possibilité de pratiquer des espaces libres en périphérie de l'implant doit être envisagée comme une autre variante possible du dispositif selon l'invention, à un autre égard, intéressant sur le plan clinique. Il est possible de pratiquer des évidements 28 partiels dans les zones extérieure et moyenne de l'implant, situées entre sa partie périphérique et l'optique, et cela en respectant le trajet des fentes, maintenues intégralement ou partiellement. L'implant ne sera plus en l'occurrence constitué d'un disque plein, sans hiatus autre que les fentes, mais d'un disque partiellement ajouré. L'intérêt potentiel de cet ajourement partiel est double. Il allège d'autant le poids de l'implant, - à vrai dire assez minime et parfaitement acceptable pour le sac capsulaire, comparativement au poids du cristallin naturel -. Surtout, il permet peut-être de mieux s'opposer à la migration centripète des cellules recouvrant et opacifiant secondairement la capsule postérieure, puisque un simple fil tendu sur cette capsule constitue un barrage efficace contre la progression des amas cellulaires. Or, ces cellules pourraient plus facilement se glisser en regard d'un implant monobloc lisse, même s'il occupe tout le sac et a une face postérieure convexe se moulant sur la capsule. Elles seraient peut-être arrêtées plus efficacement par un élément de petit diamètre, disposé circulairement 29, à un diamètre d'environ 7 à 8 mm, à peu près à mi-chemin entre un anneau périphérique 30 restant quant à lui suffisamment rigide, large et stable (- non pas, donc, une simple anse souple -) et l'optique centrale, ces deux éléments étant reliés par des ponts 31, incluant les berges 32 des fentes-guides, ponts qui pourraient être disposés comme sur la figure 4, ou de toute autre manière, et qui auraient comme point commun de maintenir à l'ensemble de l'implant une cohérence, une morphologie et une tenue d'ensemble analogues à celles d'un implant rigide. Tout en permettant donc, dans le même temps, la constitution d'éléments ajourés, demi-périphériques, où s'accumuleront les cellules veant de l'équateur du sac, - comme on l'observe dans divers cas cliniques -.

De nombreuses autres variantes du dispositif selon l'invention portent sur l'optique, dont le diamètre peut varier entre 3,5 et 7, sans exclusive dans un sens ou l'autre, - en allant même jusqu'à 10, les fentes étant alors taillées au travers de sa partie périphérique, mais aussi empiétant sur sa zone centrale, pour diminuer l'ouverture -. La forme de cette optique peut être autre que circulaire, et notamment ovale, carrée, rectangulaire ou polygonale, etc. La dimension de l'implant lui-même n'est pas limitée à 9 ou 10 mm de diamètre et peut être inférieure ou supérieure, pouvant aller notamment jusqu'à 8 ou 12. De même sa forme n'est-elle pas nécessairement circulaire, mais éventuellement ovale ou polygonale. Enfin, si l'ensemble de l'implant peut être globalement plan, (-mis à part l'optique centrale, éventuellement convexe, ou concave, etc. -), peut également être pratiquée une angulation 33 de l'ensemble de l'haptique ou seulement de sa partie périphérique, par rapport à l'optique, pour obtenir une meilleure stabilité dans l'oeil ou mieux tendre encore la capsule postérieure. Un ou plusieurs trous de positionnement peuvent aussi être pratiqués, pour faciliter les manoeuvres, mais on peut déjà se servir des fentes, en utilisant un banal habituel micro-crochet.

L'exécution du dispositif selon l'invention doit tendre à la plus grande perfection possible sur le plan technique et notamment dans le choix des matériaux, qui peuvent être de même nature pour l'optique ou l'haptique, ou bien différents. La variété des matéraux transparents envisageables est grande :verre, polysulfones, nombreux polymères, ou tous autres matériaux synthétiques déjà utilisés ou étant le fait d'inventions futures. Le polymère de loin le plus employé depuis le début de l'implantation est le polyméthylméthacrylate de méthyle (PMMA). Par rapport à lui, le verre a l'avantage d'être plus facile à stériliser. Il est plus lourd mais a un indice supérieur permettant de réaliser des lentilles plus minces mais aussi plus élaborées, par exemple avec puissance gravée, tout en ayant une bonne finition. Quel qu'il soit, le matériau transparent en question doit être par ailleurs bien toléré par l'oeil, et usinable, de façon à obtenir des lentilles de puissance bien définie dans une gamme étendue de dioptries avec progressions de l'ordre du quart ou de la demi-dioptrie, pour faire face à tous les besoins, ces lentilles pouvant être convexes, cas le plus habituel (bi-convexes, plan-convexes, ménisquées, ...), mais aussi concaves, toriques ..., avec correction dite progressive pour la vision associée de près, avec un élément recouvrant protecteur pour une meilleure biocompatibilité, avec interposition d'une lamelle intérieure, d'air par exemple, pour recherche d'une meilleure iséiconie (reconstitution d'une image la plus proche possible de celle reçue par l'oeil normal), etc. Tout l'ensemble étant conçu et réalisé selon la qualité maximale des fabrications actuelles, dans le cadre d'une miniaturisation correspondant à la chirurgie de l'oeil qui est une micro-chirurgie.

En synthèse, la présente invention apporte un progrès au domaine de la chirurgie oculaire. Il va de soi qu'elle n'a été décrite et représentée qu'à titre indicatif et explicatif mais nullement limitatif et qu'elle est susceptible de diverses variantes et modifications, dont quelques-unes seulement ont été envisagées, sans sortir de son cadre.

Notamment, il va de soi que ce qu'on a appelé la partie optique de l'implant peut occuper la plus grande partie de celui-ci, la partie haptique se réduisant à une zone périphérique de très faible dimension. Dans ce cas, les fentes sont ménagées également dans la partie optique. Cependant, la partie optique de l'implant comporte nécessairement une zone centrale dépourvue de toute fente.

## Revendications

**1 -** Implant porteur d'une lentille correctrice intra-oculaire, destiné à être introduit dans le sac capsulaire du cristallin sans déformation au travers d'ouvertures beaucoup plus petites que lui, de type notamment disque rigide, comportant une partie optique interne (23), une partie externe haptique (19), et caractérisé en ce qu'il présente des fentes ouvertes sur son bord périphérique et allant ainsi déliminter sur l'implant des secteurs dont la partie la plus grande ne dépassera pas la dimension de l'ouverture qui sera pratiquée pour son introduction dans l'oeil.

**2 -** Implant selon la revendication 1, caractérisé en ce que, tout en restant rigide, il comporte en outre des évidements partiels (28) dans sa partie haptique, évidements respectant au moins en partie le trajet des fentes.

**3 -** Implant selon la revendication 1, caractérisé en ce qu'il présente deux fentes (17-21), (26-27), ouvertes sur son bord périphérique et se dirigeant l'une et l'autre vers le centre et la partie optique, l'une de ces fentes étant plus longue (17) et développant un trajet équivalent globalement à un peu plus d'un demi-cadran horaire, l'autre plus courte (21) rejoignant plus rapidement la zone optique, la distance entre la partie la plus interne de chacune des 2 fentes et l'extrémité externe de l'autre étant égale ou inférieure à la dimension de l'incision d'ouverture de l'oeil.

**4 -** Implant selon la revendication 1, caractérisé en ce qu'il comporte deux fentes (24-25), ouvertes sur son bord périphérique, une plus longue (24) allant jusqu'à l'optique, et une plus courte (25) s'arrêtant à mi-distance de l'optique et du bord de l'implant.

**5 -** Implant selon la revendication 1, caractérisé en ce qu'il comporte plus de deux fentes (33-34-35-36-37), se développant à partir de son bord périphérique mais aussi directement à partir d'une fente elle-même, et selon alors un trajet orienté aussi bien vers le centre (35), qu'au contraire divergent (36).

**6 -** Implant selon la revendication 2, caractérisé en ce que, de part et d'autre des évidements (28) de la partie haptique, des ponts rigides (31) relient la partie périphérique annulaire rigide de l'haptique (30) à l'optique centrale, une autre partie également rigide et globalement circulaire (29) étant facultativement maintenue à la partie moyenne de l'haptique.

**7 -** Implant selon l'une quelconque des revendications 1 à 6, caractérisé en ce que la zone optique centrale est circulaire, en forme d'ovale ou en forme de polygone.
